# DEMANDE DE BREVET EUROPEEN

(11) **EP 1 745 794 A2**
(43) Date de publication de la demande: **24.01.2007**
(21) Numéro de dépôt: 06356097.3
(22) Date de dépôt: 21.07.2006
(51) Int. Cl.: A61K 35/00, A23L 1/053

(54) **Utilisation de micro-algues pour traiter et/ou prevenir les infections parasitaires**

(30) Priorité: 22.07.2005 FR 0507826
(71) Demandeur: Université Blaise Pascal, 63006 Clermont-Ferrand Cedex (FR)
(72) Inventeur: Vivares, Christian Paul, 63000 Clermont-Ferrand (FR); El Alaoui Lasmaili, Hicham, 63100 Clermont-Ferrand (FR)
(74) Mandataire: Colombet, Alain André

(57) **Abrégé**

Utilisation d'une micro-algue et/ou un extrait de micro-algue ou leurs mélanges pour la préparation d'un médicament ou comme additif alimentaire pour traiter et/ou prévenir les infections parasitaires à protozoaire.

## Description

La présente invention concerne l'utilisation de micro-algues et/ou leurs extraits pour la préparation de médicaments pour traiter et/ou prévenir les infections parasitaires à protozoaires.

Les parasitoses ou infections parasitaires humaines sont plus particulièrement répandues dans les régions chaudes du globe et/ou dans les pays pauvres mais s'étendent également à toutes les parties du monde avec l'émergence du SIDA, sous la forme de parasitoses opportunistes.

L'infection par le VIH entraîne une diminution des lymphocytes T CD4+ ou T auxiliaires et donc de l'immunité, permettant ainsi aux maladies opportunistes de se développer. Les principaux micro-organismes associés au SIDA incluent des champignons, des bactéries, des virus et des protozoaires, notamment les cryptosporidies, microsporidies et le toxoplasme. Généralement, les parasitoses opportunistes de type toxoplasmose se développent lorsque le nombre de T CD4+ est inférieur à 100/mm³.

Les toxoplasmes sont des parasites intracellulaires obligatoires responsables d'infections mortelles chez les immunodéficients. Ils ont pour cible de nombreux tissus où ils peuvent provoquer de nombreuses lésions. Il n'existe pas pour l'heure de traitements efficaces de ces parasitoses. Les difficultés à développer des thérapies sont liées à la complexité des cycles biologiques de ces protozoaires qui comportent de multiples phases faisant intervenir des organismes de caractéristiques biologiques et de localisations tissulaires variées.

Les coccidies sont des parasites unicellulaires du phylum des Apicomplexa de la classe des sporozoaires. *Toxoplasma gondii* est un parasite du type coccidie dont l'hôte primaire est le chat. Il est un parasite capable d'infester une large gamme de mammifères et d'oiseaux. Il s'agit d'un protozoaire intracellulaire obligatoire qui peut causer de la mortalité au sein des espèces humaine et animale.

Dans la nature, la contamination par *Toxoplasma* donne généralement des infections asymptomatiques. Les lésions dues à la toxoplasmose sont habituellement associées à une déficience immunitaire.

L'Homme, en temps qu'hôte intermédiaire, est contaminé par l'ingestion d'oocystes du chat, ou par la consommation de viande contaminée. L'éventail des manifestations cliniques dépend de l'âge et de la condition immunologique du patient. Pour le foetus, les conséquences de cette transmission sont très graves. Elle provoque en effet des choriorétinites, des hydrocéphalies, des calcifications cérébrales et des anomalies mentales.

Par ailleurs, 10 à 80 % des malades du SIDA développent une encéphalite très grave due à la reviviscence des kystes musculaires et cérébraux. Chez tous les patients immunodéficients, l'infection par le toxoplasme est presque toujours localisée dans le cerveau. Tous les types de cellules du cerveau peuvent être atteintes, il arrive plus rarement que les poumons et les yeux soient également infectés.

Du fait de sa très rapide réactivation, la toxoplasmose requiert un traitement immédiat efficace, et qui doit être poursuivi tout au long de la vie du patient infecté.

Les différentes molécules connues pour le traitement de la toxoplasmose incluent les macrolides, les inhibiteurs de la synthèse des purvines et pyrimidines, les inhibiteurs des voies métaboliques de l'acide folique. Il n'y a pas de molécules, non toxiques pour l'Homme, à effet parasitocide vis-à-vis des kystes, alors que 70 % de la population européenne les porte en permanence.

De nombreuses molécules à activité antiparasitaire ont déjà été étudiées mais ne traitent souvent que les symptômes et n'éradiquent pas les parasites. Les présents inventeurs ont recherché des compositions antiparasitaires ayant une activité parasitocide efficace et un minimum de toxicité cellulaire.

Par ailleurs, les parasitoses touchent également les animaux, notamment les animaux d'élevage, posant alors un problème économique important. Il en est ainsi des coccidioses ou encore, par exemple, de l'histomonose. Les coccidioses sont un grave problème en pathologie animale. Il en est ainsi de la toxoplasmose chez les ovins (avortements) et des eimerioses pour la volaille (poulet, dinde principalement), le bétail ou la cuniculture. Il est à noter que le cycle de développement, la physiologie et le métabolisme des coccidies appartenant aux genres *Toxoplasma* et *Eimeria* sont similaires.

Les coccidioses causent des pertes économiques considérables dans l'industrie aviaire (US$ 750 millions aux USA). Le poulet est sensible à une douzaines d'espèces dont les plus communes sont : *Eimeria tenella,* (diarrhée sanguinolente caecale), *E. necatrix* (diarrhée sanguinolente intestinale) et *E. acervulina* ainsi que *E. maxima* (coccidiose chronique intesinale). Pour la dinde, et la caille, le nombre est réduit respectivement à 7 et 4. Les espèces en cause pour la dinde sont *E. meleagrimitis* (localisée dans le jéjunum) et *E. adenoeides* (localisée dans l'iléon inférieur, les caeca et le rectum). La coccidiose apparaît habituellement dans les oiseaux en croissance et les jeunes adultes. Les animaux infectés consomment moins d'alimentation et d'eau, ont la diarrhée, et peuvent devenir émaciés et déshydratés. Les pondeuses présentent une réduction du taux de production d'oeufs. La coccidiose caecale peut produire des saignements et l'anémie qui est souvent suivie de la mort. La coccidiose intestinale peut être confondue avec le syndrome hémorragique d'anémie et d'autres maladies entériques. Il est difficile, sinon impossible, d'empêcher la coccidiose par la seule hygiène. Il est nécessaire d'ajouter une drogue coccidiostatique à l'alimentation qui prévient la croissance des coccidies dans la région digestive. Les coccidioses demeurent un problème important en aviculture malgré les molécules efficaces présentes sur le marché de la maladie (en raison du problème de chimiorésistance) et le lancement sur le marché d'un vaccin atténué (Paracox). Le problème augmente en raison de l'évolution de la législation européenne au sujet de l'utilisation des additifs et de la pression des consommateurs

Les coccidioses du bétail sont dues à une quinzaine d'espèces du genre *Eimeria.* Les plus fortement pathogènes sont les espèces: *E. bovis* et *E. zuernii* et plus occasionnellement *E. auburnensis.* Parasites intestinaux (intestin grêle, caecum, colon), elles causent de graves diarrhées qui sont hémorragiques dans le cas où *E. zuemii* est impliquée. Les animaux sensibles sont surtout les jeunes veaux âgés de 1 à 6 mois mais les individus plus âgés (1 à 2 ans) peuvent également être affectés. Les pertes annuelles US sont de l'ordre de US$ 100 millions.

Les coccidioses du lapin sont également importantes. Elles sont dues principalement à *E. intestinalis* et *E. flavescens* (site intestinal), et à *E.stiedai* (site hépatique). Les diarrhées entraînent des pertes de poids et une mortalité.

L'histomonose est appelée aussi maladie de la tête noire ("Black-head"). C'est une maladie parasitaire affectant les galliformes. L'agent responsable est un parasite eucaryote unicellulaire classé parmi les flagellés, *Histomonas meleagridis.* Ce parasite infecte les oiseaux d'intérêt économique comme les volailles d'élevage, et particulièrement les dindes et les perdrix. Le flagellé parasite les caecums et le foie entraînant une mortalité parfois très importante. En France, les productions de volailles les plus touchées sont les dindes. Les interdictions progressives des molécules permettant le traitement de cette maladie comme les nitroimidazoles (le dimétridazole, ronidazole...) et les additifs alimentaires comme le nifursol, appartenant à la famille des nitrofuranes ont rendu la situation difficile. En outre, les anticoccidiens comme la roxarsone (dérivé arsenical) et les antibiotiques actuellement autorisés sur le marché ou encore les benzimidazoles, comme l'albendazole ne sont pas efficaces contre *Histomonas* (Callait et al., Poult Sci. 2002, 81:1122-7 et Hegngi et al., Vet Parasitol. 1999,81:29-37). Le seul moyen de prévention efficace à ce jour reste donc la prophylaxie sanitaire (séparation des espèces, vermifuge contre *Heterakis*...). Il est donc nécessaire d'identifier des molécules d'origine végétale pour pouvoir mettre au point de nouvelles stratégies de lutte.

Le terme micro-algue désigne un organisme unicellulaire qui possède des chloroplastes et qui est donc capable de transformer l'énergie lumineuse en énergie chimique pour son développement; ce terme désigne habituellement les algues unicellulaires et les cyanobactéries.

Les algues sont des organismes eucaryotes, chlorophylliens, autotrophes ou hétérotrophes. La plupart requièrent de la lumière et du CO₂ comme sources d'énergie et du carbone. Elles sont photoautotrophes. Mais certaines sont chimiohétérotrophes et nécessitent une source de composés organiques externes.

Les végétaux en général sont connus pour avoir des métabolismes secondaires très diversifiés dont les produits sont d'un intérêt industriel majeur. Les micro-algues ont développé des voies métaboliques qui mènent à l'accumulation, parfois remarquable, de certaines substances : caroténoïdes et autres pigments, glycérol, proline, polysaccharides et acides gras.

De nombreuses espèces de micro-algues ont montré des activités antibiotiques et antifongiques (Jones, Biochesmistry of the Algae and cyanobacteria, 275-281, Proceedings of the Phytochemical Society of Europe, 1988, Clarendon, Press.).

Les présents inventeurs ont découvert que des micro-algues et/ou leurs extraits présentaient une activité antiparasitaire contre différents protozoaires.

Selon un premier objet, la présente invention concerne donc l'utilisation de micro-algues et/ou leurs extraits pour la préparation d'un médicament ou additif alimentaire pour prévenir et/ou traiter les infections parasitaires à protozoaires.

Selon un premier aspect, ladite micro-algue est choisie parmi les algues unicellulaires et/ou leurs extraits.

Selon un aspect préféré, les micro-algues sont choisies parmi les genres: *Rhodella, Cosmarium, Kirchneriella, Closterium, Coelastrum, Isochrysis ;* plus préférentiellement, les micro-algues selon l'invention sont choisies parmi les espèces *Rhodella violacea, Cosmarium* sp, *Kirchneriella lunaris, Closterium* sp, *Isochrysis galbana.*

On préfère tout particulièrement l'espèce *Kirchneriella lunaris.*

Le genre *Rhodella* fait partie de la classe des *Rhodophyceae,* de l'ordre des *Porphyridales* et de la famille des *Porphyridaceae.*

Aucune activité microbicide n'avait encore été démontrée.

*Cosmarium* et *Kirchneriella* font partie des Chlorophycées.

Le genre *Cosmarium* fait partie de la classe des Zygophycées, de l'ordre des Desmidiales.

Le genre *Kirchneriella* fait partie de l'ordre des Chlorococcales et de la famille des Oocystaceae. Cette espèce se présente sous forme unicellulaire ou sous forme de colonies.

Aucune activité microbicide n'avait été encore démontrée.

*Closterium* fait partie des Zygnematales.

Aucune activité microbicide n'avait été encore démontrée.

Selon un deuxième aspect, ladite micro-algue est une cyanobactérie ou un extrait de cyanobactérie. Il peut notamment s'agir d'un cyanobactérie appartenant au genre *Chroococcidiopsis,* e.g. l'espèce *C. thermalis.*

Selon un autre aspect de la présente invention, lesdites infections parasitaires sont choisies parmi les infections coccidiennes.

Selon un premier aspect préféré, ladite infection coccidienne est due à un parasite du genre *Toxoplasma,* de préférence *Toxoplasma gondii.*

Plus particulièrement, ladite infection coccidienne est la toxoplasmose.

Préférentiellement, la micro-algue est *Kirchneriella lunaris* et/ou ses extraits et ladite infection coccidienne est la toxoplasmose.

Selon une première réalisation, ledit médicament est destiné aux patients immunodéprimés, en particulier suite à une infection par le VIH (virus de l'immunodéficience humaine).

Selon une seconde réalisation, ledit médicament est destiné aux mammifères femelles en gestation, notamment les femmes enceintes ou les ovins en gestation.

Selon un autre aspect de la présente invention, ladite infection parasitaire est l'histomonose; l'histomonose est due à un parasite du genre *Histomonas,* et plus particulièrement l'espèce *Histomonas meleagridis.*

Pour le traitement et/ou la prévention de l'histomonose, on préfère utiliser *Kirchneriella lunaris* et/ou des extraits de *Kirchneriella lunaris.*

Selon un aspect avantageux de l'invention, ledit médicament est destiné à traiter et/ou prévenir l'histomonose chez l'espèce aviaire. On peut notamment citer la dinde, la pintade, la perdrix, le canard, la poule (de chair ou pondeuse), le poulet ; plus préférentiellement la dinde.

Etant donné la similitude de physiologie et de métabolisme entre *Toxoplasma* et *Eimeria,* l'activité anti-toxoplasmose des extraits de micro-algue est prédictive de l'activité anti-coccidioses prophylactique et/ou thérapeutique chez les animaux d'élevage (volaille, bétail, lapin).

Selon un autre aspect de la présente invention, ladite infection coccidienne est due à un parasite du genre *Eimeria,* de préférence *Eimeria tenella, Eimeria necatrix, Eimeria acervulina, Eimeria maxima* chez le poulet, *Eimeria meleagrimitis, Eimeria adenoeides* chez la dinde et la caille, *Eimeria bovis* et *Eimeria zuernii* chez le bétail, ou encore *Eimeria intestinalis, Eimeria flavescens* et *Eimeria stiedai* chez le lapin.

Plus particulièrement, ladite infection coccidienne est l'eimériose.

Préférentiellement, la micro-algue est *Kirchneriella lunaris* et/ou ses extraits et ladite infection coccidienne est la l'eimériose.

Les extraits de micro-algue selon l'invention désignent tout extrait de micro-algue ou les mélanges de ces extraits, les extraits étant obtenus par extraction avec un solvant approprié, selon les méthodes d'extraction connues en soi. A titre de solvant, tout solvant habituellement utilisé pour l'extraction de végétaux peut convenir. On préfère notamment utiliser comme solvant l'eau, les alcools, tels que le méthanol ou l'éthanol, le chloroforme.

De préférence, les extraits obtenus ont des concentrations comprises entre 0,1 et 10 %, notamment entre 0,1 et 5%.

Les extraits peuvent être concentrés par des méthodes classiques. On peut ainsi produire des extraits ayant une concentration en micro-algue de l'ordre de plusieurs unités à plusieurs dizaines de %, par exemple de 5 à 60%.

Selon un autre objet, la présente invention concerne également un médicament pour traiter et/ou prévenir les infections parasitaires à protozoaire comprenant une micro-algue et/ou un extrait de micro-algue et un véhicule acceptable sur le plan pharmaceutique ou vétérinaire.

Selon un autre objet, la présente invention concerne également un additif alimentaire pour traiter et/ou prévenir les infections parasitaires à protozoaire comprenant une micro-algue et/ou un extrait de micro-algue, notamment chez les animaux d'élevage.

Selon un autre objet, la présente invention concerne en outre une association de micro-algues et/ou leurs extraits pour traiter et/ou prévenir les infections parasitaires à protozoaire. Plus préférentiellement, les associations selon l'invention contiennent 2, 3 ou 4 micro-algues choisies parmi *Rhodella violacea, Cosmarium* sp, *Kirchneriella lunaris, Closterium* sp et/ou leurs extraits.

Selon un autre aspect, la présente invention concerne une méthode de prévention et/ou traitement d'infections parasitaires à protozoaire comprenant l'administration d'une micro-algue et/ou un extrait de micro-algue et un excipient acceptable sur le plan pharmaceutique ou vétérinaire, à un patient qui en a besoin.

Les médicaments selon l'invention comprennent une micro-algue et/ou extrait et/ou leurs associations avec un excipient acceptable sur le plan pharmaceutique ou vétérinaire.

De préférence, ladite composition contient une quantité efficace de la micro-algue et/ou extrait et/ou leurs associations. De préférence, ledit médicament est administré à un patient qui en a besoin.

Les compositions pharmaceutiques ou vétérinaires selon l'invention peuvent être présentées sous des formes destinées à l'administration par voie parentérale, orale, rectale, permuqueuse ou percutanée.

Elles seront donc présentées sous forme de solutés ou de suspensions injectables ou flacons multi-doses, sous forme de comprimés nus ou enrobés, de dragées, de capsules, de gélules, de pilules, de cachets, de poudres, de suppositoires ou de capsules rectales, de solutions ou de suspensions, pour l'usage percutané dans un solvant polaire, pour l'usage permuqueux.

Les excipients qui conviennent pour de telles administrations sont les dérivés de la cellulose ou de la cellulose microcristalline, les carbonates alcalino-terreux, le phosphate de magnésium, les amidons, les amidons modifiés, le lactose pour les formes solides.

Lorsqu'ils sont utilisés à titre d'additif alimentaires, les extraits ou leurs mélanges sont ajoutés directement à l'alimentation, par exemple à l'eau de boisson.

Pour l'usage rectal, le beurre de cacao ou les stéarates de polyéthylèneglycol sont les excipients préférés.

Pour l'usage parentéral, l'eau, les solutés aqueux, le sérum physiologique, les solutés isotoniques sont les véhicules les plus commodément utilisés.

La posologie peut varier dans les limites importantes (0,5 mg à 1000 mg) en fonction de l'indication vétérinaire ou thérapeutique et de la voie d'administration, ainsi que de l'âge et du poids du sujet.

Le terme patient utilisé ici désigne les humains ou animaux, notamment les volailles.

La présente invention peut être illustrée de façon non limitative par les exemples suivants pris en liaison avec les figures suivantes :
- la figure 1 est un graphe montrant l'incorporation d'uracile tritiée par *T. gondii* en présence ou pas d'un extrait aqueux de *K. lunaris à* des concentrations variables ;
- la figure 2 est un graphe montrant l'incorporation d'uracile tritiée par *T. gondii* en présence ou pas d'un extrait aqueux de *R. violacea* à des concentrations variables ;
- la figure 3 est un graphe montrant l'incorporation d'uracile tritiée par *T. gondii* en présence ou pas d'un extrait aqueux de *Cosmarium sp* à des concentrations variables ;
- la figure 4 est un graphe montrant l'activité des extraits chloroformiques de certaines micro-algues sur *T. gondii* ;
- la figure 5 est un graphe montrant la croissance *d'Histomonas meleagridis* dans le milieu Stepkowski ;
- la figure 6 est un graphe montrant l'activité des extraits chloroformés de micro-algues sur *Histomonas meleagridis* (72 heures de culture) ;
- la figure 7 est un graphe montrant l'activité d'extraits de micro-algues sur *T. gondii.*

### Exemples - Partie 1

### 1. Matériel biologique

Les espèces micro-algues utilisées sont les suivantes :
- *Rhodella violacea* (Algothèque UTEX), Rhodophycée (n° LB2427).
- *Cosmarium* sp. Chlorophycée (n°145 de la collection du Muséum d'Histoire Naturelle de Paris)
- *Kirchneriella lunaris* (Algothèque UTEX), Chlorophycée (n° 285).
- *Kirchneriella lunaris* (Algothèque SAG, Sammlung von Algenkulturen, Gôttingen, Allemagne), Chlorophycée (n° SAG243-1).
- *Closterium* sp. Zygnematale (n°100 de la collection du Muséum d'Histoire Naturelle de Paris)

### 2. Méthodes

### 3.1. Milieux de culture

### 3.1.1. Micro-algues d'eau douce

Les micro-algues d'eau douce sont cultivées dans du milieu Protéose dont la préparation est la suivante : pour 940 mL d'eau distillée, ajouter 1 g de peptone de protéose, et les constituants suivants:

| mL | stock solution | g/400 mL H₂O |
|---|---|---|
| 10 | NaNO₃ | 10,0 |
| 10 | CaCl₂·2H₂O | 1,0 |
| 10 | MgSO₄·7H₂O | 3,0 |
| 10 | K₂HPO₄ | 3,0 |
| 10 | KH₂PO₄ | 7,0 |
| 10 | NaCl | 1,0 |

### 3.1.2. Micro-algues marines

Pour les micro-algues marines, un autre milieu, milieu ES est utilisé:

| | |
|---|---|
| NaNO₃ | 350 mg |
| Na₂glycerophosphate 5H₂O | 50 mg |
| **Solution de Fer** | 25 mL |
| **Métaux PII** | 25 mL |
| vitamin B₁₂ | 10 µg |
| Thiamine | 0,5 mg |
| Biotin | 5 µg |
| Tampon Tris (Sigma Co.) | 500 mg |

Ajuster le pH à 7,8, dispenser en tubes (20 mL/tube) et autoclaver. Stocker à 10°C.
**Solution de Fer.** Dissoudre 351 mg de Fe(NH₄)₂(SO₄)₂ · 6H₂O et 300 mg de Na₂EDTA dans 500 mL d'eau distillée.
**Solution de métaux PII.** Pour 100 mL d'eau distillée, ajouter:

| | |
|---|---|
| Na₂EDTA | 100.0 mg |
| H₃BO₃ | 114,0 mg |
| FeCl₃·6H₂O | 4,9 mg |
| MnSO₄·H₂O | 16,4 mg |
| ZnSO₄·7H₂O | 2,2 mg |
| CoSO₄·7H₂O | 0,48 mg |

Pour un milieu prêt à l'emploi, ajouter 20 mL de milieu ES à de l'eau de mer autoclavée pour un volume total d'1 L.

### 3.2. Préparation des extraits aqueux, méthanoliques ou chloroformiques de micro-algues

Une fois la culture prélevée, une centrifugation est réalisée pendant 5 minutes à 200 g. Le culot est ensuite repris dans 1 mL d'eau stérile, de méthanol ou de chloroforme. Le mélange est ensuite vortexé pour les culots repris dans du chloroforme ou broyé à l'aide d'un potter pour les culots repris dans de l'eau ou dans du méthanol, cette étape est réalisée en chambre froide (4°C), cette étape est réalisée deux fois pendant 5 minutes. Il est ensuite mis sous agitation pendant une nuit à 4°C.

### 3.3. Activité antitoxoplasmose

*Toxoplasma* est choisi par les présents inventeurs comme modèle de coccidie.

### 3.3.1. Cytotoxicité

La cytotoxicité des extraits est réalisée sur les cellules MRC5, cellules humaines hôtes pour la coccidie *Toxoplasma gondii,* ceci permet de vérifier l'innocuité des extraits vis-à-vis de la cellule hôte permettant d'observer des effets spécifiques contre le parasite. Cette cytotoxicité est évaluée selon la méthode à la sulforhodamine B (SRB) qui est un colorant qui se lie aux protéines et dont l'adsorption peut être mesurée au spectrophotomètre à 550 nm (coloration rose).

Pour ce test ont été cultivées à 37°C, des cellules MRC5 (Biomérieux) dans une plaque 96 puits (Costar) à raison de 2.10⁴ cellules par puits dans 200 µl de milieu MEM (Gibco) auquel a été ajouté 5% de sérum de veau foetal. Après 24h de culture, le milieu est changé et les extraits dont on veut tester la toxicité sont ajoutés après avoir été repris dans le volume de solvant approprié indiqué (diméthylsulfoxide, après avoir vérifier au préalable que les concentrations en DMSO n'étaient pas toxiques pour les cellules, ou eau). Les concentrations indiquées dans les tableaux sont les concentrations finales dans les puits. On laisse 48h en culture. Fixer ensuite les cellules en ajoutant 50 µL de TCA (acide trichloroacétique) froid par puits, à la surface du milieu de culture. Laisser reposer la plaque 5 minutes à température ambiante puis 2 heures à 4°C.

Le surnageant est ensuite éliminé et les puits sont lavés 5 fois avec 100 µL d'eau (la plaque étant vidée par retournement). Laisser alors sécher la plaque. Elle peut être conservée sèche jusqu'à coloration.

100 µL de sulforhodamine B (à 0.4% dans de l'acide acétique 1 %) sont ajoutés dans chaque puits. La coloration dure 20 minutes. La sulforhodamine est ensuite enlevée et les puits rincés 5 fois à l'acide acétique 1 %. Solubiliser alors la coloration liée aux cellules pendant 5 minutes en ajoutant 100 µL de Tris Base 10 mM (pH 10.5).

La densité optique est lue à 550 nm sur un lecteur de microplaques (Bio-Rad model 550).

### 3.3.2. Quantification du développement de Toxoplasma gondii par mesure de l'activité β-Galactosidase

La souche de *Toxoplasma gondii* utilisée correspond à la souche RH recombinante, un gène codant pour la β-Galactosidase ayant été introduit dans le génome du parasite.

Des cellules MRC5 confluentes dans des plaques 96 puits sont infestées par 2.10⁴ tachyzoites (200 µL) pendant 1 heure. Celles-ci sont lavées une fois avec du milieu MEM supplémenté. Les extraits à tester sont ensuite ajoutés à la bonne concentration dans un volume de 200 µL.

Deux témoins sont réalisés: cellules saines et cellules infestées non traitées.

Après lyse des cellules par le parasite (48 à 72 heures), les plaques sont centrifugées à 200 g pendant 5 minutes à température ambiante. Les parasites sont lysés avec 50 µL de tampon de lyse 1 heure à 50°C.

| ***Tampon de lyse*** | |
|---|---|
| 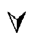 HEPES | 100 mM pH 8 |
| 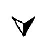 MgSO₄ | 1 mM |
| 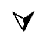 Triton X 100 | 1 % |
| 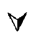 DTT | 5 mM |

La lyse est vérifiée au microscope, et il faut ajouter ensuite 160 µL de tampon de réaction et laisser agir 5 minutes à 37°C.

| ***Tampon de réaction*** | |
|---|---|
| 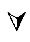 Tampon phosphate | 100 mM pH 7.3 |
| 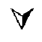 β mercaptoéthanol | 102 mM |
| 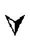 MgCl₂ | 9 Mm |

40 µL de chlorophénolred-β-D-galactopyranoside, substrat de la β-Galactosidase présente dans le parasite, à une concentration de 6,25 mM en solution dans du tampon phosphate pH 7,3 est ajouté dans les puits.

| ***Tampon phosphate 0.1 M pH 7.3*** | |
|---|---|
| 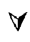 Na₂HPO₄ | 1 M |
| 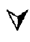 NaH₂PO₄ | 1 M |

Ajuster le pH à 7.3

| ***Tampon de révélation*** | |
|---|---|
| 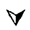 tampon phosphate | pH 7.3 |
| 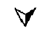 chlorophénolred- β-D-galactopyranoside | 6.25 mM |

La réaction se fait à 37°C jusqu'à obtenir une coloration rouge, la DO à 540 nm (+ grande sensibilité à 570 nm) / 420 nm est mesurée, celle-ci traduit la quantité de β-Galactosidase présente dans les puits, ce qui révèle donc la quantité de parasité présent dans les puits.

### 3.3.3. ELISA

### Culture :

1- Mettre en culture les plaques 96 puits dans du milieu MEM supplémenté (200 µL par puit).
2-A la confluence des cellules, infester par 2.10⁴ tachyzoites de *T.gondii* pendant 1 heure.
   Réaliser 2 témoins :
   ↔ témoin cellules saines
   ↔ témoin cellules infestées
3- Ajouter les extraits végétaux à tester lors du changement de milieu après infestation.
4-Après lyse des cellules (6-7 jours), fixer les puits par 100 µL de méthanol froid pendant une nuit à - 80°C (ou 30 min -20°C)

### ELISA :

Le test est basé sur la reconnaissance spécifique d'un anticorps des protéines parasitaires de surface de *T. gondii,* cela permet donc d'évaluer la croissance du parasite.
1- Laver les plaques avec 100 µL de PBS 1X.
2- Saturer par de la BSA 1 % diluée dans du PBS 1X.
   Saturer 2 heures sous agitation à température ambiante.
3- Oter la BSA 1 % et ajouter 50 µL d'Ac primaire polyclonal anti-toxoplasme dilué au 1/1000^{ième} dans du PBS 1X - Tween 20 0.1%.
   Incuber les plaques 2 heures à température ambiante
4- Eliminer l'excès d'Ac par 3 lavages rapides (5 min) au PBS 1X ― Tween 0.1% (100 µL) puis ajouter 50 µL d'Ac secondaire anti-lg de lapin couplé à la phosphatase alcaline dilué au 1/10 000^{ième}.
   Incuber les plaques 2 heures à température ambiante
5- Après incubation, laver 3 fois rapidement les plaques avec du PBS 1X - Tween 0.1% (100 µL) puis révéler au moyen du para-nitro-phénylphosphate (pNPP) à 1 mg/mL (100 µL) dans du tampon diéthanolamine 0.5M pH 9.8 pendant 30 minutes à 37 °C à l'obscurité.
6- Stopper la réaction par ajout de 150 µL de soude 3M puis mesurer la DO à 405 nm à l'aide du lecteur de microplaques *Biorad* model 550.

### 3.3.4. Incorporation d'uracile tritiée

Cette technique est basée sur l'incorporation sélective d'uracile tritiée par *Toxoplasma gondii.* En effet, seul le parasite possède l'enzyme, l'uracile phosphoribosyltransférase, capable de convertir l'uracile en uridine. En incubant avec de l'uracile tritiée, seule l'activité métabolique du parasite est observée.

### Résultats

L'ELISA permet d'observer la quantité de parasites présents, l'incorporation d'uracile tritiée, l'activité métabolique spécifique du parasite. Chaque point est le résultat de 3 expériences indépendantes. Il avait été vérifié au préalable que les concentrations utilisées n'étaient pas toxiques pour les cellules hôtes.

### 1. Kirchneriella lunaris (extrait aqueux)

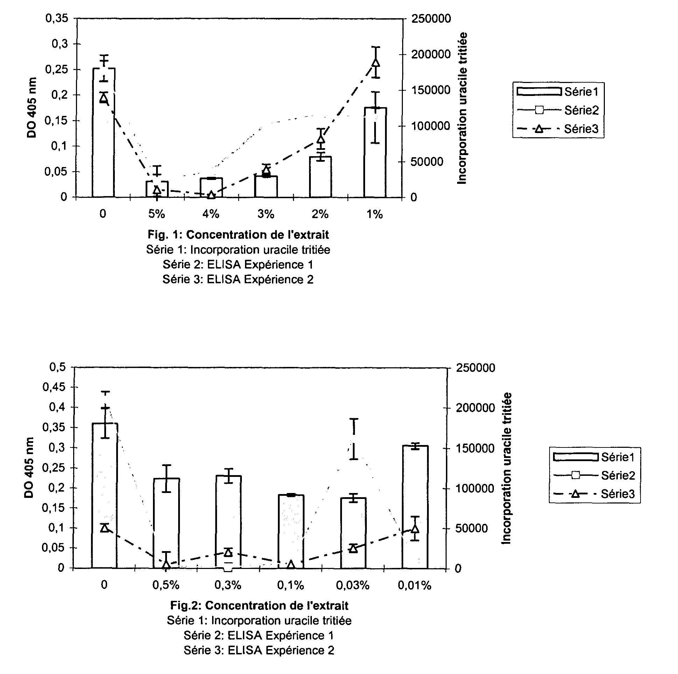

Les résultats sont illustrés à la Figure 1. Pour *K. lunaris,* un effet dose-réponse est obtenu ; à 5% de produit, l'inhibition du parasite est totale (100% d'inhibition du parasite). Ceci est démontré par 3 séries d'expériences distinctes : 2 en ELISA et 1 par incorporation d'uracile tritiée.

### 2. Rhodella violacea (extrait méthanolique)

Les résultats sont illustrés à la Figure 2. L'effet le plus important est observé à 0,1% dans les 3 séries d'expériences distinctes : 2 en ELISA et 1 par incorporation d'uracile tritiée.

### 3 Cosmarium sp (extrait méthanolique)

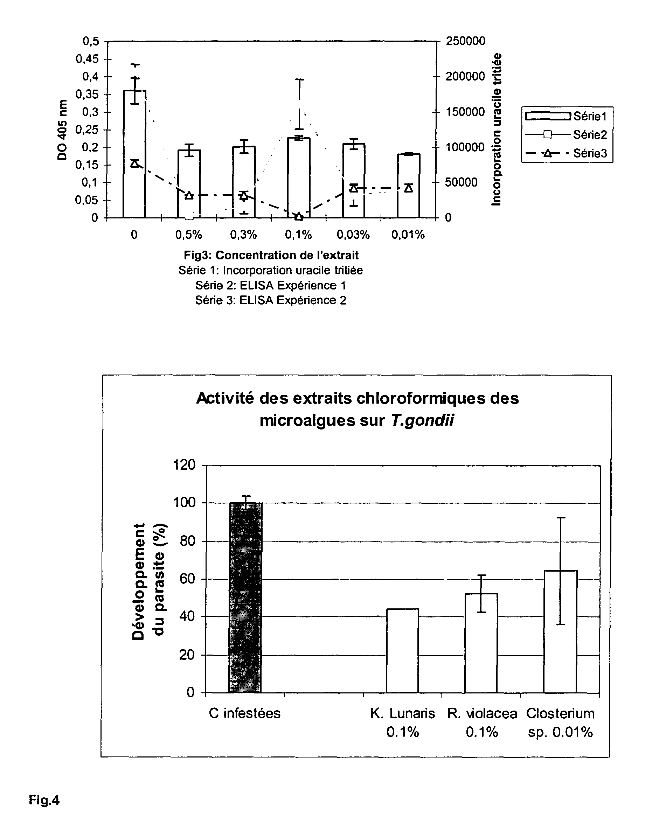

Les résultats sont illustrés à la Figure 3. L'effet le plus important est observé à la concentration la plus forte (0,5%) dans les 3 séries d'expériences distinctes : 2 en ELISA et 1 par incorporation d'uracile tritiée.

### 4 Extraits chloroformiques

Des extraits chloroformiques des 3 micro-algues précédemment citées, plus d'autres ont été testés ; ces résultats ont été obtenus par la technique à la β-galactosidase.

On obtient environ 60% d'inhibition du parasite pour *K. lunaris,* 50% pour *R. violacea,* et environ 40% pour *Closterium.* Les résultats sont illustrés à la Figure 4.

Les traits verticaux aux figures 1-4 correspondent aux écarts-types.

### 3.4. Activité anti-histomonose

### 3.4.1. Culture du parasite

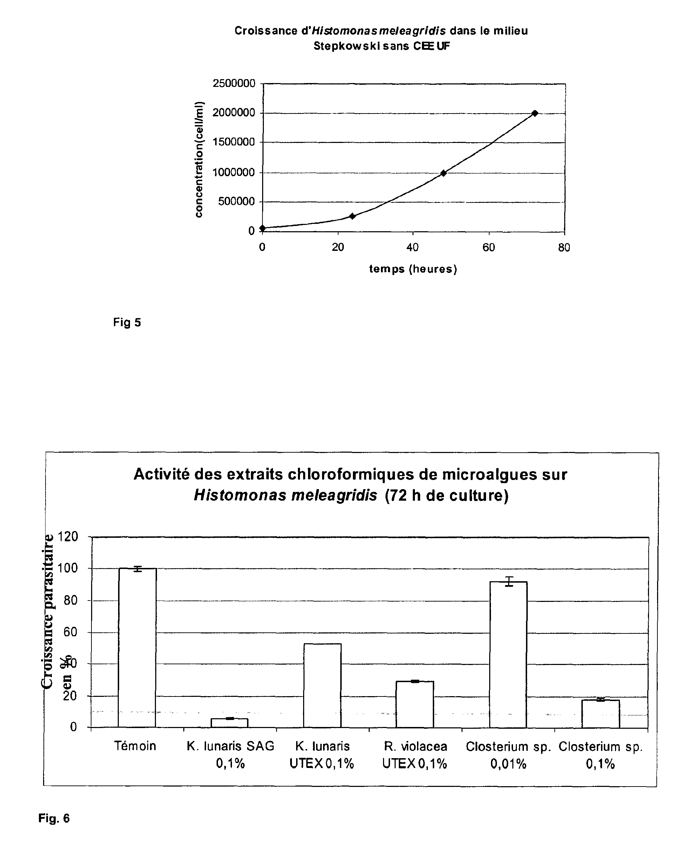

Le parasite *H. meleagridis* est cultivé traditionnellement à 39°C dans un milieu mis en place par Stepkowski en 1895 et repris par Dwyer en 1970 contenant du milieu 199 (80 à 85%),de l'extrait embryonnaire de poulet (5 à 10%), du sérum de cheval (5%) et de l'amidon de riz (5%). Cette culture est qualifiée d'agnotobiotique, c'est à dire il y a présence d'une flore bactérienne non déterminée issue du caecum de dinde. L'observation de cette flore en microscopie photonique indique qu'elle comprend différents types bactériens. L'extrait embryonnaire de poulet a été éliminé. Le parasite se développe alors très bien avec un facteur de multiplication de 40 au bout de 72 heures (voir figure 5).

### 3.4.2. Criblage des extraits

Un protocole a été mis au point pour tester l'efficacité histomonicide *in vitro* de différents extraits. Les formulations sont ajoutées à différentes concentrations dans le milieu de culture. A chaque test, le milieu est ensemencé avec 5.10⁴ *Histomonas.* Après 72H de culture à 39°C, les parasites sont observés au microscope photonique et sont comptés à l'aide d'une cellule de Thoma (à chaque fois 10 champs sont comptés et la moyenne effectuée). De plus, des tests de cytotoxicité sur des cellules de mammifères (HFF) ont été effectués sur les extraits pour déterminer la toxicité sur cellules de mammifères. Les concentrations utilisées sont non cytotoxiques.

### 3.4.3. Résultats

Les résultats sont illustrés à la figure 6.

L'extrait chloroformique de *K. lunaris* issu de l'algothèque SAG (Sammlung von Algenkulturen, Göttingen, Allemagne) présente la plus forte activité (plus de 90% d'inhibition) contre *H. meleagridis* ainsi que l'extrait chloroformique de *Closterium* sp. (plus de 80%). Pour *Closterium* nous avons testé deux concentrations et nous avons obtenu un effet dose-réponse puisqu'à 0,01 % il n'y a plus d'activité. L'extrait chloroformique de *R. violacea* présente une activité antiparasitaire de l'ordre de 70%.

### Exemples - Partie 2

### 1. Essais in vitro

Des extraits aqueux ont été préparés (comme décrit dans Exemples - Partie 1, 3.2.) à partir d'une micro-algue du genre *Coelastrum* (Classe des Chlorophycées) et de l'espèce *lsochrysis galbana* (Classe des Haptophycées), un extrait chloroformique a été obtenu à partir de l'espèce *Chroococcidiopsis thermalis* (Cyanobactéries). Ces micro-algues ont été obtenues du Laboratoire de Cryptogamie, Muséum d'Histoire Naturelle, Paris, France, où elles ont identifiées sous les références respectivement n° 162, n° 688 et n° 443. Les extraits ont été testés sur *T. gondii* par mesure de l'activité β-galactosidase selon le protocole standard présenté dans Exemples - Partie 1, 3.3.2.

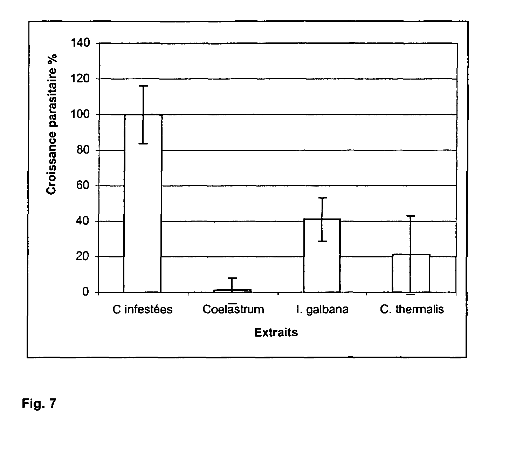

Ces résultats sont présentés à la Figure 7. C. infestées = cellules MRC5 infestées par *Toxoplasma gondii.*

Les extraits issus de *C. thermalis* et du genre *Coelastrum* ont montré des activités anticoccidiennes respectivement de 80 % et de presque 100 %, l'extrait issu de *I. galbana* présente une inhibition de 60 % de la croissance parasitaire.

### 2. Essais in vivo concernant les extraits chloroformiques de Kirchneriella lunaris et Rhodella violacea préparés comme décrit dans Exemples - Partie 1, 3.2.

### 2.1. Protocole

Les tests d'efficacité de coccidiostats ont été réalisés en dispositifs de 18 cages à raison de 7 poulets/cage. Ce dispositif permet de comparer 6 lots avec 3 répétitions de chaque lot. Nous comparons 4 lots traités à 2 lots témoins : un lot non inoculé non traité (NINT) et un lot inoculé non traité (INT).

Le protocole d'essai a été réalisé comme suit :
J0 : arrivée de 200 poussins en batterie chaude
J12 : baguage, pesée, histogramme des poids, rejet des extrêmes pour conserver 126 poulets de poids homogènes qui sont répartis 7/cage dans les 18 cages.

### Début des traitements

J19 : pesées, inoculation des coccidies et essais des produits
J26 : pesées, autopsie de 3 poulets/cage
J27 : contrôle de l'excrétion d'oocystes

### Critères étudiés

Les critères parasitologiques étudiés sont la mortalité, les scores lésionnels et les excrétions d'oocystes sur la période J25-27.

Les critères zootechniques sont le poids et le gain de poids, le calcul du GMQ (gain moyen quotidien), de la consommation d'aliment et de l'indice de conversion.

### 2.2. Résultats

Les essais thérapeutiques de coccidiostatiques se sont bien déroulés selon le protocole préétabli. Les deux produits biologiques testés (Extrait de *Kirchneriella lunaris* et extrait de *Rhodella violacea)* ont donné des résultats très satisfaisants.

Les animaux ont gardé un appétit normal et croissant. Ceci s'est de facto répercuté sur leur poids corporel, donc leur croissance. Leurs performances ne sont pas très éloignées de celles des témoins NINT. Par contre, les témoins INT ont fait la maladie avec des mortalités importantes et une baisse considérable de leur consommation alimentaire. Aucune mortalité n'a été attribuée aux produits testés ce qui permet de confirmer leur caractère non toxique.

En conclusion, au terme de cette série d'essais *in vivo,* les extraits expérimentés comme coccidiostatiques sur les poulets de chair sont effectivement doués de propriétés coccidistatiques tout en étant non toxiques pour les poulets.

## Revendications

1. Utilisation d'une micro-algue et/ou un extrait de micro-algue ou leurs mélanges pour la préparation d'un médicament ou comme additif alimentaire pour traiter et/ou prévenir les infections parasitaires à protozoaire chez l'homme ou les animaux.

2. Utilisation selon la revendication 1 pour laquelle ladite micro-algue est choisie parmi les algues unicellulaires et/ou leurs extraits.

3. Utilisation selon la revendication 1 ou 2 pour laquelle ladite micro-algue est choisie parmi les espèces des genres: *Rhodella, Cosmarium, Kirchneriella, Closterium, Coelastrum, Isochrysis,* et/ou leurs extraits.

4. Utilisation selon l'une quelconque des revendications 1 à 3 pour laquelle ladite micro-algue est une cyanobactérie, de préférence du genre *Chroococcidiopsis,* notamment *Chroococcidiopsis thermalis.*

5. Utilisation selon l'une quelconque des revendications précédentes pour laquelle ladite micro-algue est choisie parmi *Rhodella violacea, Cosmarium* sp, *Kirchneriella lunaris, Closterium* sp, *Isochrysis galbana, Chroococcidiopsis thermalis* et/ou leurs extraits.

6. Utilisation selon l'une quelconque des revendications précédentes pour laquelle ladite micro-algue est du genre *Kirchneriella* et/ou ses extraits.

7. Utilisation selon l'une quelconque des revendications précédentes pour laquelle ladite micro-algue est *Kirchneriella lunaris* et/ou ses extraits.

8. Utilisation selon la revendication 7 pour laquelle les extraits de *Kirchneriella lunaris* sont des extraits chloroformiques ou aqueux.

9. Utilisation selon l'une quelconque des revendications précédentes pour laquelle lesdites infections parasitaires sont choisies parmi les infections coccidiennes.

10. Utilisation selon l'une quelconque des revendications précédentes pour laquelle ladite infection coccidienne est due à un parasite du genre *Toxoplasma.*

11. Utilisation selon la revendication 10 pour laquelle ladite infection coccidienne est due au parasite *Toxoplasma gondii.*

12. Utilisation selon la revendication 10 ou 11 pour laquelle ladite infection coccidienne est la toxoplasmose.

13. Utilisation selon l'une quelconque des revendications précédentes pour laquelle la micro-algue est *Kirchneriella lunaris* et/ou ses extraits et ladite infection coccidienne est la toxoplasmose.

14. Utilisation selon l'une quelconque des revendications précédentes pour laquelle ledit médicament est destiné aux patients atteints du VIH.

15. Utilisation selon l'une quelconque des revendications 1 à 9 pour laquelle ladite infection parasitaire est l'histomonose.

16. Utilisation selon la revendication 15 pour laquelle l'histomonose est due à un parasite du genre *Histomonas.*

17. Utilisation selon la revendication 16 pour laquelle l'histomonose est due au parasite *Histomonas meleagridis.*

18. Utilisation selon l'une quelconque des revendications 15 à 17 pour laquelle la micro-algue est *Kirchneriella lunaris.*

19. Utilisation selon l'une quelconque des revendications 15 à 18 pour laquelle ledit médicament ou additif alimentaire est destiné aux volailles.

20. Utilisation selon l'une quelconque des revendications 1 à 9 pour laquelle ladite infection parasitaire est l'eimériose.

21. Utilisation selon la revendication 20 pour laquelle l'éimériose est due à un parasite du genre *Eimeria tenella, tenella, Eimeria necatrix, Eimeria acervulina, Eimeria maxima, Eimeria meleagrimitis, Eimeria adenoeides, Eimeria bovis* et *Eimeria zuernii, Eimeria intestinalis, Eimeria flavescens* et *Eimeria stiedai.*

22. Utilisation selon l'une quelconque des revendications 20 à 21 pour laquelle la micro-algue est *Kirchneriella lunaris.*

23. Utilisation selon l'une quelconque des revendications 20 à 22 pour laquelle ledit médicament est destiné aux volailles, ovins, lapins.

24. Médicament pour traiter et/ou prévenir les infections parasitaires à protozoaire comprenant une micro-algue et/ou un extrait de micro-algue ou leurs mélanges et un véhicule acceptable sur le plan pharmaceutique ou vétérinaire

25. Médicament selon la revendication 24 pour lequel les infections parasitaires à protozoaire sont telles que définies en revendications 9, 10, 11, 12, 15, 16 ou 17.

26. Médicament selon la revendication 24 ou 25 pour lequel la micro-algue et/ou extrait est telle que définie en revendications 2 à 8.

27. Additif alimentaire pour traiter et/ou prévenir les infections parasitaires à protozoaire comprenant une micro-algue et/ou un extrait de micro-algue ou leurs mélanges.

28. Additif alimentaire selon la revendication 27 pour lequel les infections parasitaires à protozoaire sont telles que définies en revendications 9, 10, 11, 12, 14, 15, 16, 17, 20, 21.

29. Additif alimentaire selon la revendication 27 ou 28 pour lequel la micro-algue et/ou extrait est telle que définie en revendications 2 à 8.

30. Additif alimentaire selon la revendication 28 ou 29 pour administration aux animaux.

31. Association de micro-algues et/ou leurs extraits pour traiter et/ou prévenir les infections parasitaires à protozoaire.

32. Association selon la revendication 31 pour laquelle les micro-algues sont choisies parmi *Rhodella violacea, Cosmarium* sp, *Kirchneriella lunaris, Closterium* sp, *Coelastrum, Isochrysis galbana, Chroococcidiopsis thermalis* et/ou leurs extraits.
